Europäisches Patentamt

European Patent Office

Office européen des brevets

(11) Veröffentlichungsnummer: **0 053 688**
**B1**

# (12) EUROPÄISCHE PATENTSCHRIFT

(45) Veröffentlichungstag der Patentschrift:
28.09.83

(51) Int. Cl.³: **C 07 C  45/50,** C 07 C  27/22,
C 07 C  29/16, B 01 J  31/28

(21) Anmeldenummer: **81108694.1**

(22) Anmeldetag: **22.10.81**

(54) **Verfahren zur Hydroformylierung von olefinisch ungesättigten Verbindungen.**

(30) Priorität: **09.12.80  DE 3046355**

(43) Veröffentlichungstag der Anmeldung:
**16.06.82 Patentblatt 82/24**

(45) Bekanntmachung des Hinweises auf die Patenterteilung:
**28.09.83 Patentblatt 83/39**

(84) Benannte Vertragsstaaten:
**AT BE DE FR GB IT NL SE**

(56) Entgegenhaltungen:
**EP-A-0 017 183**

(73) Patentinhaber: **BASF Aktiengesellschaft,
Carl-Bosch-Strasse 38, D-6700 Ludwigshafen (DE)**

(72) Erfinder: **Richter, Wolfgang, Dr., Osloer Weg 19,
D-6700 Ludwigshafen (DE)**
Erfinder: **Kummer, Rudolf, Dr., Kreuzstrasse 6,
D-6710 Frankenthal (DE)**
Erfinder: **Schwirten, Kurt, Dr., Prager Strasse 27,
D-6700 Ludwigshafen (DE)**

0 053 688

## Verfahren zur Hydroformylierung von olefinisch ungesättigten Verbindungen

Die vorliegende Erfindung betrifft ein verbessertes Verfahren zur Hydroformylierung von olefinisch ungesättigten Verbindungen (im folgenden auch als »Olefine« bezeichnet) mittels aus Rhodiumacetat in situ hergestellten Rhodium/Triphenylphosphin/Carbonyl-Komplexen.

Dieses Verfahren, sieht man von der erfindungsgemäßen Verbesserung ab, ist aus zahlreichen Veröffentlichungen allgemein bekannt und bedarf daher keiner näheren Erläuterung.

Die erfindungsgemäße Verbesserung beruht auf der Beobachtung, daß sich der aktive Komplex bei Einsatz von Rhodiumacetat und Triphenylphosphin unter den Bedingungen der Synthese relativ langsam bildet. Da der Olefinumsatz mit der Menge des aktiven Katalysators zunimmt, bedeutet dies, daß der volle Umsatz erst nach einer Betriebszeit von einigen Tagen erreicht wird. Verwendet man dagegen von Anfang an den fertigen Komplex H · Rh(CO)(PPh$_3$)$_3$ (Ph = Phenyl), so tritt eine derartige Induktionsperiode nicht auf.

Der Erfindung lag daher die Aufgabe zugrunde, die Induktionsperiode bei der Hydroformylierung von Olefinen mittels Rh/PPh$_3$/CO-Komplexen, die aus Rh-Acetat und PPh$_3$ in situ gebildet werden, zu unterdrücken.

Demgemäß wurde gefunden, daß keine Induktionsperiode bei der Hydroformylierung von olefinisch ungesättigten Verbindungen mittels in situ aus Rhodiumacetat, Triphenylphosphin und Kohlenmonoxid gebildeten Komplexen auftritt, wenn man der Hydroformylierungsreaktion eine Startphase vorschaltet, bei der das Gemisch aus dem Hydroformylierungsmedium, dem Triphenylphosphin und Rhodiumacetat unter einem CO/H$_2$-Druck von 5 – 20 bar so lange auf 90 – 120° C erhitzt und die freigesetzte Essigsäure in einem CO/H$_2$-Strom gasförmig aus dem Reaktor ausgetragen wird, bis in diesem gasförmigen Austrag praktisch keine Essigsäure mehr nachweisbar ist.

Diese Startphase, die lediglich etwa 2 – 8 Stunden in Anspruch nimmt, läßt sich kurz gesagt auch dadurch charakterisieren, daß sie mit Ausnahme der Olefinzufuhr alle Maßnahmen der Hydroformylierung umfaßt. Hieraus läßt sich schließen, daß die Bildung des aktiven Katalysatorkomplexes überraschenderweise begünstigt wird, wenn kein Olefin zugegen ist.

Das Hydroformylierungsmedium besteht zweckmäßigerweise aus hochsiedenden Hydroformylierungsnebenprodukten wie Estern, Acetalen und Aldolkondensationsprodukten.

Da sich derartige Produkte in der Startphase noch nicht gebildet haben können, muß man auf solche früherer oder paralleler Produktionen zurückgreifen. Sind solche Produkte nicht verfügbar, kann man auch sonstige höhersiedende inerte Lösungsmittel wie Diole und Diolester, z. B. 2,2,4-Trimethylpentan-1,3-diol-monoisobutyrat verwenden. 1 Liter des Hydroformylierungsmediums enthält ferner in der Regel 20 – 120 g Triphenylphosphin sowie 100 – 500 mg Rhodium, und zwar zunächst in salzartiger und später in koordinativer Bindung. Ist alles Rhodium in Komplexform umgewandelt, lassen sich die Olefine unter einem Druck eines etwa äquimolaren CO/H$_2$-Gemisches von 5 – 30 bar bei 80 – 150° C zu den entsprechenden Aldehyden bzw. Gemischen der entsprechenden isomeren Aldehyde umsetzen.

Da die an die erfindungsgemäße Katalysatoraktivierung anschließende Hydroformylierung keine Rückwirkung auf diese Aktivierung hat, kommt es auf die Art der Olefine bei der Hydroformylierungsreaktion naturgemäß nicht an, d. h., das Verfahren eignet sich als Vorstufe für die Hydroformylierung beliebiger Olefine. Besondere Bedeutung hat es im Falle der Hydroformylierung von Ethylen, Propylen sowie den C$_8$ – C$_{12}$-Alkenen.

Die als Verfahrensprodukte entstehenden Aldehyde sowie daneben auch Alkohole können wie üblich zusammen mit CO, H$_2$ und dem nicht umgesetzten Olefin gasförmig aus dem Reaktor entnommen werden. Nach Kondensation und Abtrennung der Aldehyde werden das Olefin sowie der Großteil von CO und H$_2$ wieder als sogenanntes Kreisgas in den Reaktor zurückgeführt.

Diese Kreisgasfahrweise wird auch während der erfindungsgemäßen Startphase praktiziert, mit dem Unterschied, daß hier statt der Aldehyde und des Olefins der Alkohol, der von der alkoholischen Rh-Acetatlösung stammt, sowie vor allem die freigesetzte Essigsäure ausgetragen werden.

Handelt es sich um höhersiedende Olefine und damit auch höhersiedende Aldehyde, so kann die Kreisgasfahrweise wegen des relativ geringen Partialdrucks dieser Verbindungen weniger zweckmäßig sein als die Entnahme des flüssigen Reaktionsgemisches aus dem Reaktor und die hierauf folgende übliche Aufarbeitung. In diesem Fall wendet man die Kreisgasfahrweise nur in der Startphase an.

Als Alkohole zur Bereitung der Rh-Acetatlösungen eignen sich vornehmlich Methanol und Ethanol. Die Konzentration des Rh-Acetats in diesen Lösungen beträgt vorzugsweise 1 – 10 Gew.-%.

Die während der erfindungsgemäßen Komplexbildung abgespaltene und mit dem Kreisgas ausgetragene Essigsäure läßt sich im Kreisgas wie üblich, z. B. gaschromatographisch, nachweisen.


Beispiel

Mit Hilfe einer Versuchsapparatur, die zu 60 Vol.-% mit einem Gemisch aus 95 Gew.-% hochsiedender Hydroformylierungsprodukte und 5 Gew.-% Triphenylphosphin gefüllt war, wurde die Anfangsperiode der Propylen-Hydroformylierung mittels Rh-Katalysatoren untersucht, wobei der Propylenumsatz für die folgenden Bedingungen ermittelt wurde:

— A (zum Vergleich): das Rh wurde als 5gew.-%ige methanolische Lösung von Rh-Acetat eingesetzt und lag zu Beginn der Hydroformylierung im Reaktor vor.

CO/$H_2$-Verhältnis (molar): 1 : 1
Gesamtdruck: 15 bar
Temperatur: 110°C
Rh-Konzentration: 150 mg/kg Reaktionsmedium
Propylenzufuhr: 60 g/h/l Reaktionsmedium

— B (zum Vergleich): Das Rh wurde als 5gew.-%ige toluolische Lösung des Komplexes H · Rh(CO)(PPh$_3$)$_3$ eingesetzt und lag zu Beginn der Hydroformylierung im Reaktor vor. Reaktionsbedingungen wie unter (A).

— C (erfindungsgemäß): Das Rh wurde wie unter (A) eingesetzt, wonach das Gemisch unter den Bedingungen von (A), jedoch ohne Propylenzuführung, der Rh-Komplexbildung (Startphase) unterworfen wurde. Nach 4,5 Stunden war keine Essigsäure im Abgas mehr nachweisbar, so daß anschließend die Hydroformylierung gemäß (A) vorgenommen wurde.

Die Ergebnisse der Versuche (A) — (C) sind der folgenden Tabelle zu entnehmen.

| Betriebszeit t (h) (ohne Startphase) | Propylenumsatz nach t Stunden (%) | | |
|---|---|---|---|
| | A | B | C |
| 2 | 58 | 95 | 89 |
| 6 | 63 | 93 | 90 |
| 12 | 75 | 96 | 90 |
| 24 | 82 | 95 | 92 |
| 48 | 84 | 94 | 92 |
| 96 | 85 | 92 | 91 |

Aus den Versuchsergebnissen folgt, daß der Katalysator im Falle A selbst nach 4 Tagen noch nicht die volle Aktivität von rd. 90% erreicht hatte wie im Falle B oder C.

Die Verwendung des fertigen Rh-Komplexes (Fall B) bietet zwar den Vorteil einer hohen Aktivität von Anfang an, die zu Beginn sogar noch etwas größer ist als im Falle C, erfordert dafür aber auch die umständliche gesonderte Bereitung des Rh-Komplexes. Verglichen damit ist die Arbeitsweise gemäß Versuch C wesentlich wirtschaftlicher.

**Patentanspruch**

Verfahren zur Hydroformylierung von olefinisch ungesättigten Verbindungen mittels in situ aus Rhodiumacetat, Triphenylphosphin und Kohlenmonoxid gebildeten Rhodium/Triphenylphosphin/Carbonyl-Komplexen, dadurch gekennzeichnet, daß man der Hydroformylierungsreaktion eine Startphase vorschaltet, bei der das Gemisch aus dem Hydroformylierungsmedium, dem Triphenylphosphin und Rhodiumacetat unter einem CO/$H_2$-Druck von 5 — 20 bar so lange auf 90 — 120°C erhitzt und die freigesetzte Essigsäure in einem CO/$H_2$-Strom gasförmig aus dem Reaktor ausgetragen wird, bis in diesem gasförmigen Austrag praktisch keine Essigsäure mehr nachweisbar ist.

**Claim**

A process for hydroformylating olefinically unsaturated compounds by means of rhodium/triphenylphosphine/carbonyl complexes formed in situ from rhodium acetate, triphenylphosphine and carbon

3

**0 053 688**

monoxide, wherein the hydroformylation reaction is preceded by a starting phase in which the mixture of the hydroformylation medium, the triphenylphosphine and rhodium acetate is heated at from 90 to 120°C under a CO/$H_2$ pressure of from 5 to 20 bar and the acetic acid liberated is discharged from the reactor in gaseous form in a stream fo CO and $H_2$, until virtually no more acetic acid can be detected in this gaseous discharge.

## Revendication

Procédé pour l'hydroformylation de composés insaturés oléfiniquement au moyen de complexes de rhodium/triphénylphosphine/carbonyle formés in situ à partir d'acétate de rhodium, le triphénylphosphine et d'oxyde de carbone, caractérisé en ce qu'on fait précéder la réaction d'hydroformylation d'une phase initiale, dans laquelle le mélange du milieu d'hydroformylation, de la triphénylphosphine et de l'acétate de rhodium est chauffé à 90 – 120°C, sous une pression de CO/$H_2$ de 5 à 20 bars, et l'acide acétique libéré est évacué du réacteur à l'état gazeux dans un courant de CO/$H_2$, jusqu'à ce qu'on ne puisse pratiquement plus déceler d'acide acétique dans cette décharge gazeuse.

4